# EUROPEAN PATENT SPECIFICATION

(11) **EP 1 071 385 B1**
(45) Date of publication and mention of the grant of the patent: **15.10.2003**
(21) Application number: 99915924.7
(22) Date of filing: 12.04.1999
(51) Int. Cl.: A61F 5/01

(54) **APPENDAGE CORRECTION DEVICE**
KORREKTURVORRICHTUNG FÜR KÖRPERTEILE
DISPOSITIF DE CORRECTION D'APPENDICES

(30) Priority: 15.04.1998 GB 9807858; 13.11.1998 GB 9824807
(43) Date of publication of application: 31.01.2001
(73) Proprietor: Dynamic Healthcare Ltd., Blackburn Waterside, Blackburn BB1 5BL (GB)
(72) Inventor: FOOTE, Brian, Oswaldtwistle, Lancashire BB5 3PN (GB)
(74) Representative: Denmark, James Christopher
(86) International application number: GB9901120
(87) International publication number: WO99052477

(56) References cited:
- EP-A- 0 625 341
- FR-A- 484 521
- US-A- 3 815 587
- US-A- 4 456 002
- US-A- 4 790 300
- US-A- 5 382 224
- US-A- 5 653 680

## Description

This invention relates to an appendage correction device, in particular to an appendage correction device which is spring biased for correcting the attitude of a particular appendage of a human or mammal with respect to the particular portion of that human or mammal to which said appendage is attached. Examples include wrists, feet or other limbs, or one or more of a plurality of digits, or optionally the head of a human or mammal which are all prone to adopt incorrect or deficient attitudes.

Although the following description relates almost exclusively to the provision of a spring biased device such as a dynamic splint for correcting the attitude of the fingers of a human which may be clawed as a result of hypertension, a stroke, fit or other debilitating condition, it will be appreciated by persons skilled in the art that the invention has application in the correction of any portion of the body which without a constantly applied correcting force adopts an unconventional position. Examples include dropped foot and head, clawed fingers and toes, etc.

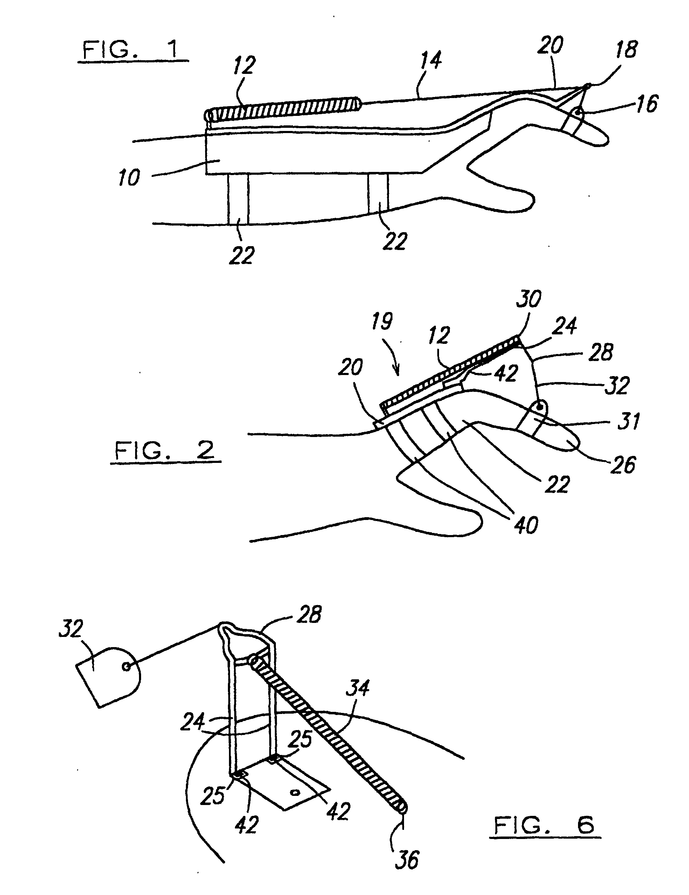

A previous type of splint for extending a user's fingers from a clawed position is shown in Figure 1. This type of splint comprised a rigid back portion 10 to be secured to the back of a user's hand and along their wrist. The back portion 10 is typically made of plastic and is pre-formed to follow the shape of a user's hand and wrist.

One end of a spring 12 is secured at the end of the back portion 10 closest to the user's elbow. The other end of the spring is secured to a piece of fishing line 14, which itself is secured to a sling 16 placed over the user's finger. The fishing line 14 is run over a pulley 18 which is secured to a raised lip 20 at the end of the back portion 10 closest to the user's fingers. The length of the spring 12 and fishing line 14 are arranged so that tension is exerted on the user's finger to straighten the finger to improve the clawing condition. The splint is secured to the user's wrist with straps 22.

The fundamental disadvantage with splints of the type described above arises from the simple physics associated with Hooke's law for the extension of springs, being ***F=kx**,* where F is the force required to extend the spring, x is the extension of the spring under the said force, and k is the spring constant. When a user attempts to grip an item by curling his fingers, he has to apply a muscular force to overcome the resistance of the spring before his fingers will curl. In accordance with Hooke's law, this force increases as the spring extends and thus renders the gripping and manipulation of items difficult, especially small items where one or more of the fingers of the user may be required to be curled through 90° such that the fingers are perpendicular to the palm of the hand or through an even greater angle.

Also, the grip cannot be maintained for any great length of time because a continuous force must be used to maintain the extension of the spring. Typically, such a device becomes painful to wear after one or two hours.

A further disadvantage of the existing splints is the gradual deterioration of their therapeutic effect as the fingers are straightened. It is most preferable for splints to exert only a nominal correcting force on the fingers at first to avoid inflicting excessive pain and discomfort caused by immediately applying a large correcting force, and thereafter steadily increase the correcting force. However, existing splints function in the entirely opposite manner as they are usually first applied when the fingers are completely clawed, and the springs which apply the correcting force to the fingers are therefore at their maximum extension. Henceforth as the fingers are straightened, the spring extension reduces as does the correcting force, hence necessitating the use of a number of different springs of different lengths or spring constants which can be successively affixed to the splint and to the finger slings after the attitude of the fingers have been corrected by an appropriate amount. Only in this manner can a suitable correcting force be continually applied to the fingers.

It is commonly believed that utilisation of the deficient limbs or digits during the period of correction is therapeutic and can reduce the time taken to effect the correction. Many of the currently existing splints do not offer the facility for utilising the fingers because the wearer is dissuaded from so utilising his fingers on account of the increased force required to move same, and concomitantly the increased pain and discomfort which may be associated with such movement.

It can be appreciated from the above that existing splints are cumbersome and unwieldy, and require the continual changing of springs as the deficiency of the user is corrected. This means that the user is required to return to hospital periodically which is undesirable. Ultimately, existing splints are so troublesome to users that they are often completely removed and discarded by the user who would rather suffer the problems associated with a clawed hand than continually wear the splint. Such circumstances are completely unacceptable, and the present invention has as one of its objects the mitigation of the above disadvantages.

Furthermore, this previous type of device does not allow a user to move his wrist.

US4790300 to Marx discloses a correction device according to the first panel of claim 1. It concerns a dynamic splinting component for treating wrist conditions or injuries. In particular, there is disclosed a wrist gauntlet which is attached to the forearm of a patient, and to said gauntlet is attached a frame consisting of a spine and a channel section to which the spine is attached at one end thereof and substantially centrally of the web of said channel section. The flanges of the channel section are ideally disposed on either side of the wrist and upwardly of the spine, and a U-shaped wire is pivotally connected to the extremities of said flanges. The web of the U-shaped wire is disposed above the hand and an elastic tensioning member connected thereto and also to the gauntlet in the region thereof which surrounds the upper forearm. A palmar sling is also connected to the web of the U-shaped wire and depends therefrom and in use the weight of the hand of a user of the device disposed in the sling provides a reaction against the action of the elastic tensioning member. The pivotal connection of the U-shaped wire allows the gauntlet, via the frame, to carry the larger component of force exerted by the elastic tensioning member, whereas the lesser component is transmitted through the palmar sling to the hand of the user disposed therein. However, the construction of this device is such that the force component transmitted to the hand can never be completely eliminated.

It is an object of the invention to mitigate against the abovementioned disadvantages associated with existing slings, and to provide a dynamic splint in which the force, transmitted by the splint to the appendage being corrected thereby, can be reduced substantially to zero.

According to a first aspect of the present invention these is provided a correction device according to claim 1 and its dependent claims.

The fundamental advantage of such configuration is that when the appendage is most severely deflected away from its correct attitude, the restoring force transmitted to the appendage is minimal or zero, and therefore the user suffers least pain. Additionally, when a restoring force is transmitted to the appendage, the user is only required to exert a slight countering force to reduce the restoring force to zero. Hence, the appendage is subjected to progressively increasing forces as the attitude is corrected.

The device according to the invention has a number of advantages over currently existing splints, and these are now explained.

The component of the correcting force applied between the appendage and the portion of the user to which said appendage is attached progressively reduces and is increasingly carried by the projection member of the device as it rotates about the base portion. This is invaluable because firstly, the component of the correcting force applied to the appendage progressively increases as the projection member rotates in a direction which reduces the extension of the resilient member because as it does so, the angle between the projection member and the resilient member increases and accordingly the component of the correcting force carried by the said projection member decreases. This is desirable from a medical perspective as discussed above.

Secondly, when the projection member has rotated to such an extent that it is substantially parallel with the resilient member, practically the entire correcting force applied by the resilient member between the projection member and the base portion is carried by the projection member. The component of the correcting force transmitted to the appendage is minimised and even eliminated, and in the case of a finger splint, the user can grasp and manipulate items with relative ease. Indeed, the user can be quite dextrous when the projection member is in such an orientation.

A yet further advantage of the device is that it allows a user to exercise the deficient appendage while its attitude is being corrected, and this is widely believed to be of great physiotherapeutic benefit.

Specific embodiments of the present invention will now be described, by way of example, with reference to the accompanying drawings, in which:
Figure 1 is a schematic side view of a prior art splint;
Figure 2 is a schematic side view of a finger splint according to the present invention in use;
Figure 3 is a partial schematic side view of the finger splint of figure 2 in use;
Figure 4 is a partial schematic side view, similar to figure 3 but with the finger splint in a different position;
Figure 5 is a partial schematic side view of the finger splint in a further position;
Figure 6 is a partial schematic perspective view of the finger splint;
Figure 7 is a schematic side view of a wrist splint according to the present invention in use;
Figure 8 is a schematic top view of the wrist splint of figure 7;
Figure 9 is a schematic side view similar to figure 7, but with a wrist splint in a different position;
Figure 10 is a schematic view of an orthopaedic device for a foot in a first configuration; and
Figure 11 is a schematic view of the device shown in Figure 10, in a second configuration.

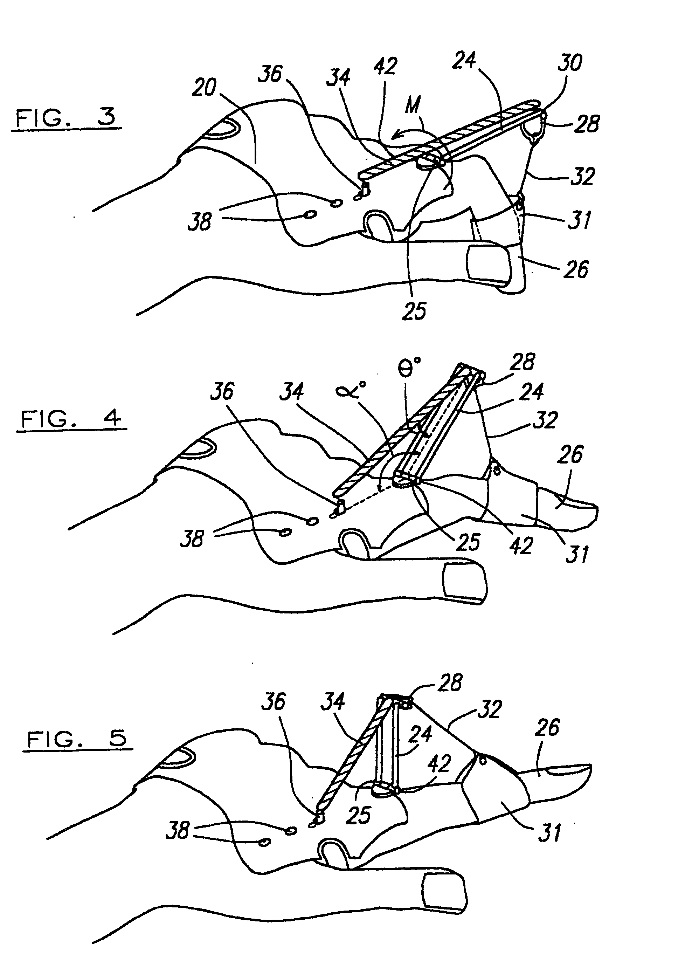

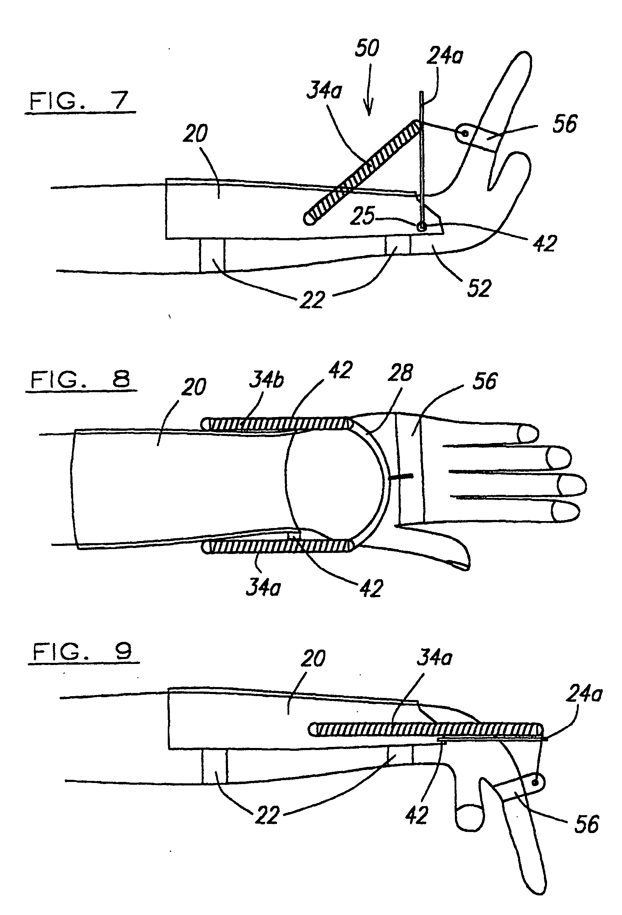

A finger splint 19 comprises a rigid back portion 20 sized to fit over the back of a patient's hand 22. A pivotally mounted pin 24 is secured to the end of the back portion 20 which is closest to the patient's fingers 26. The pin 24 is moveable between a position in which it is approximately perpendicular to the rigid back portion 20 (see figure 5) and a position in which it is approximately parallel to the rigid back portion 20 (see figure 3) . A lever 28 extends perpendicularly from the free end 30 of the pin 24. A finger sling 32 is secured to the free end of the lever 28 with a length of fishing line, wire or the like. As shown in figure 6 the pin 24 comprises twin uprights joined at the top. This configuration provides good strength and stability.

A spring 34 is adjustably secured to the rigid back portion 20 by means of a peg 36 which is inserted into one of a number of corresponding apertures 38, which are arranged longitudinally along the rigid back portion 20. The spring 34 extends past the base of the pivoted pin 24 to a free end 30 of the pin to which it is secured (see figure 3).

In use, the finger splint 19 is secured to a user's hand with straps 40, which may be fixed with a hook and pile fastener, or a buckle or the like. Alternatively Velcro® straps may be used. The rigid back portion 20 is located on the back of a user's hand 22 and the finger sling 31 is placed over one of the user's fingers 26.

When the user exerts no force on the finger sling 31 with his finger 26, the finger splint 19 ideally assumes the configuration shown in figure 5, in which the pin 24 is substantially perpendicular to the rigid back portion 20 and the spring 34 is in a contracted state. The user's condition may prevent this ideal position though. The pin 24 has a stop 25 at its pivotally mounted base 42 to prevent the pin 24 being pulled beyond the point shown in figure 5 by the spring 34.

The lengths of the pin 24, the spring 34, the lever 28 and the length of wire 32 are chosen so that in the configuration shown in figure 5 the user's finger is approximately straight. The user's finger 26 is not pulled beyond straight by virtue of the stop 25 at the base 42 of the pin 24. The lever 28 has the effect of causing the finger sling 31 to be pulled directly away from the finger 26, rather than at an acute angle to the finger 26, which would result in the finger sling 31 being pulled along the user's finger 26. The length of the lever 28 can be varied to suit different sizes of finger.

When the user moves his fingers 26, the finger sling 31 pulls the pin 24 which moves about its base 42. As the pin 24 moves the spring 34 is stretched, as shown in figure 4.

The mode of operation can be best described with reference to Figure 4.

Two factors combine to significantly reduce the correcting force which is applied to the finger 26. On the one hand, the extension of the spring 34 acts to restore the pin 24 to its original vertical position shown in Figure 5. As the finger 26 is curled further, the extension of the spring is increased and according that force acting to restore the pin 24 to its original position also increases. However, this does not automatically mean that the correcting force applied to the finger is increased because on the other hand, the angle θ° decreases, and therefore the component of the restoring force of the spring 34 transmitted through the pin 24 to the base increases. Accordingly, the force required to maintain the pin at a particular angle *α*° with respect to the base portion reduces as *α*° increases and θ^{°} reduces. An equilibrium point is reached as shown in Figure 3 whereat the force required to maintain the pin 24 at a particular angular orientation is minimised and even eliminated. As the pin rotates beyond this orientation, practically all of the restoring action of the spring 34 is transmitted through the pin 24 to the base portion 20 and only minimal force is required to overcome any restoring bending moment exerted by the spring 34 as a result of its contact over the pivot 25 as shown in Figure 3. It will be noted that although the spring 34 is substantially parallel with the pin 24 in this figure, their lines of action are spaced apart, the line of action of the spring being above the pivot 25 such that the restoring force of the spring 34 on the pin 24 gives rise to a small anticlockwise moment M about said pivot which still acts to correct the attitude of the finger 26.

Consequently, once a user has moved the pivot pin beyond the equilibrium position when gripping an object, for instance when gripping a steering wheel, the user is able to maintain the grip for considerable periods of time with having to exert undesirably large forces in holding the spring 34 in an extended configuration. The base 42 of the pin 24 has a further stop (not shown) which prevents the pin 24 from rotating too far.

In the manner described above, the finger splint 19 exerts a straightening tension on a finger inserted into the finger sling 31 whilst the finger is either straight or partially bent. When the finger 26 is bent (by roughly 80° to 90°) the tension on the finger 26 is much reduced by the interaction of the spring and the pin 24, thus allowing the user to grip an object for a reasonable period of time.

Additionally, the interaction of the spring 34 and the pin 24 results in an increase in the amount of tension on the finger 26 felt by the user as the finger 26 is moved from the position shown in figure 3 to the position shown in figure 5.

This is because the component of the restoring force of the spring carried by the pin is reduced as α° decreases and θ° increases, and therefore the force required to maintain the pin in a particular orientation increases.

The tension of the spring 34 may be adjusted by moving the peg 36 and inserting it into one of the other apertures 38, either further away from the base 42 of the pin 24 to increase the tension or nearer to the base 42 to reduce the tension.

The splint 19 described above shows only one finger sling 31. Further finger splints with corresponding springs and pins etc. could be fitted to the rigid back portion 20 to allow a number of fingers to be exercised at the same time.

The device could also be used to straighten a user's thumb, in which case a pair of pivot pins 24 and springs 34 would be provided which allow movement in two perpendicular planes. One plane could be the same as that of the finger movement described above with the other being at 90° to that to allow sideways movement also.

Figures 7 to 9 show an embodiment of a wrist splint 50, which has all the same features as the finger splint 19 described above, except that the rigid back portion 20 is secured above the user's wrist to allow movement of the wrist joint 52. The finger sling 31 is replaced by a hand sling 56 which is passed around the palm of a user's hand. Also, the wrist splint 50 is provided with two pins 24a and 24b with respective springs 34a and 34b. One of the spring and pivot pairs is located at one side of the rigid back portion 20 and the other pair is located at the other side of the rigid back portion 20. The wrist splint 50 functions in the same manner as the finger sling 31 except that it is the wrist joint of the user which is put under tension, or not as the case may be, rather than the user's finger joint.

The wrist splint 50 has the same advantages for prolonged use as the finger splint.

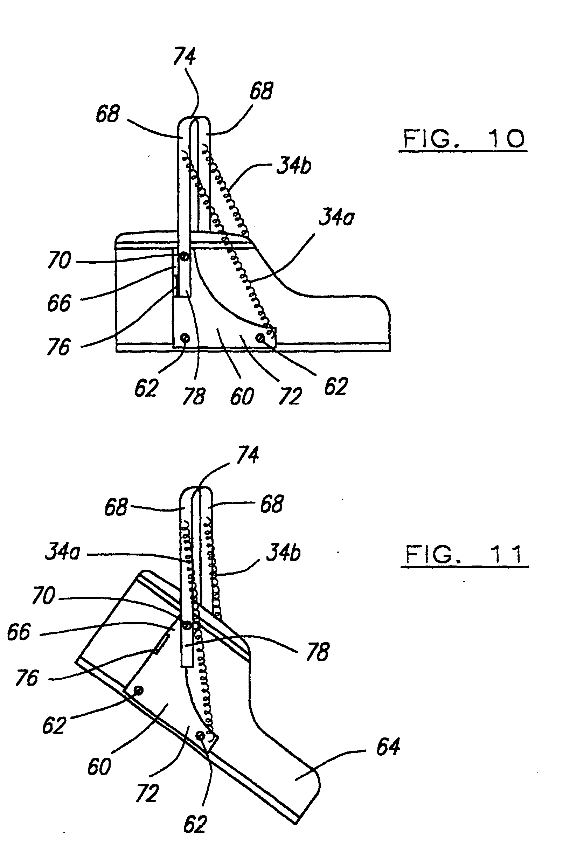

Figures 10 and 11 show a further embodiment of the present invention, in the form of an correcting device for correcting the condition known as dropped foot. In this condition, a person's foot tends to point downwards, rather than assuming the usual approximate right angle with the lower leg.

The device comprises a pair of base portions 60, being secured by screws 62 to respective sides of a shoe 64 to be worn by the user. The base portions 60 are generally "L" shaped with one limb 66 of the "L" extending up the side of the shoe 64. At the end of each limb 66 a projection member 68 (corresponding to the pivot pin 24 in the first embodiment) is secured by a pivot 70. The two projection members 68 extend roughly vertically upwards. Springs 34a and 34b are secured between an upper end of the projection members 68 and the other limb 72 of the base portion 60. The projection members 68 may be joined to each other by a transverse section 74.

The projection members 68 may move rearwardly against the force of the springs 34a and 34b, to allow a user to move their foot downwardly. Forward movement of the projection members 68 beyond the vertical (upward flexing of the foot) is prevented by stops 76, which abut lower projections 78 of the projection members 68.

The device in Figures 10 and 11 uses the same principle as the devices described above, in that when the person's foot is flexed downwards, for instance when using a car foot pedal, the tension from the springs 34a and 34b of the device is taken up by the projection members 68, as shown in Figure 11. When the user's foot is relaxed, which would in a healthy person, normally result in the foot being in a position shown in Figure 10, the springs 34a and 34b exert tension on the foot by creating tension between the base portions 60 and the pivotally projection members 68 to which the springs are secured, in order to correct the drop foot.

In this embodiment, although the intended orientation of the foot (approximately right angles to the lower leg) is different to that of the finger and wrist splints described above (a linear alignment), the principle of operation is the same. In this embodiment the base portions 60 correspond to the back portion 20 of the first embodiment and the projection members 68 of this embodiment correspond to the pins 24 of the previous embodiment. The main difference is that the base portions 60 in this embodiment are secured to the portion of the user's body to be flexed, rather than being secured to the portion of the body adjacent to the portion to be flexed, as is the case in the first and second embodiments. Nonetheless, when the foot is flexed and in use, the tension in the springs is carried increasingly by the projection members 68 as they come into substantially parallel alignment with the springs 34a and 34b.

Other embodiments of the invention could comprise orthopaedic devices for straightening, or giving support to, a user's neck, in which case the user's "appendage" would be his head.

It is to be mentioned that in all the embodiments of the invention described, the point of attachment of the resilient member to the base portion, the pivot of the pivotable projection member and the point of attachment of the resilient member to the pivotable projection member can assume a position of alignment, and in this position the resilient member is also simultaneously aligned therewith. In this position, the restoring force on the appendage whose attitude is to be corrected is practically eliminated, and the device is in a state of unstable equilibrium. Any slight rotation of the projection member from the position described results in both an increase in the restoring force applied to the appendage, and the force exerted by the resilient member acts to rotate the projection member even further away from said unstable equilibrium position towards a neutral equilibrium position shown in Figures 5, 7, 10.

## Claims

1. A correction device for correcting the attitude of an appendage of a user with respect to a portion of that user to which said appendage is attached at a joint
said device comprising
a base portion (20, 60) having means (22, 62) for securing the device to the appendage (26), or the user portion on one side of the joint,
a projection member (24, 24a, 68) pivotally attached on said base portion in a first location (25, 70) and having means (31, 56, 74) acting on the appendage or user portion on the alternate side of the joint
and a resilient member (34, 34a, 34b) having a first end attached on the base portion in a second location (36) remote from the first location (25, 70) and a second end attached to the projection member at a third location (30)
the arrangement being such that rotation of the projection member away from the second location increases the extension of the resilient member thus increasing the magnitude of a force on said projection member acting to restore said projection member while simultaneously decreasing the angle (θ°) subtended between the resilient member and the projection member at the third location so that a greater component of the restoring force is transmitted through the projection member back to the base and a lesser component of said force acts to correct the attitude of the appendage with respect to the user portion
**characterised in that**
the projection member can be rotated away from the second location to a position in which said projection member and the resilient member are aligned and the restoring force of the resilient member acting on said projection member is entirely carried by said projection member thus minimising the force acting to correct the attitude of the appendage with respect to the user portion.

2. A correcting device according to claims 1 **characterised in that** the base portion is secured to the user portion to which the appendage is attached.

3. A correcting device according to claims 1 **characterised in that** the base portion is secured to the appendage, and **in that** the projection member has means which act on the user portion to which the appendage is attached at the joint.

4. A correcting device according to claim 1 **characterised in that** the pivotal movement of the projection member away from the second location is limited by stop means to prevent further rotation of said projection member beyond the position in which it is aligned with the resilient member.

5. A correcting device according to claim 1 **characterised in that** the projection member comprises at least two upright sections which are joined at a free end thereof.

6. A correcting device according to claim 1 **characterised in that** the second end of the resilient member secured in the third location at or close to the alternate end of the projection member to that at which it is attached to the base.

7. A correcting device according to claim 1 **characterised in that** the first end of the resilient member is adjustably secured to the base portion to allow for adjustment in the tension of the resilient member.

8. A correcting device according to any preceding claim **characterised in that** the first location is proximate the joint.

9. A correcting device according to any preceding claim **characterised in that** the device comprises a plurality of projection members and associated resilient members and is applicable to a user to correct the attitudes of a plurality of fingers with respect to a hand to which said fingers are attached at knuckles.

10. A correcting device according to claim 9 **characterised in that** the projection member is provided with a sling in which a finger may be disposed.

11. A correcting device according to any of claims 1, or 3-8 **characterised in that** the projection member is provided with a sling in which a hand may be disposed, said base portion being secured to a forearm of the wearer to which said hand is attached at a wrist.

12. A correcting device according to any preceding claim **characterised in that** the resilient member is a coiled spring.

13. A correcting device according to either of claims 9-10 **characterised in that** the projection member comprises an extension section which extends from a free end thereof and away from the resilient member.

14. A correcting device according to claim 1 **characterised in that** the base portion is secured to the user with straps, which have hook and pile fasteners secured thereto.

15. A correcting device according to claim 1 **characterised in that** the device is a correction device for a foot, said base portion being secured to the foot attached to the leg of a wearer at the ankle and the projection member being pivotally attached to the base to act on said leg to correct the attitude of said foot with respect to said leg.

16. A correcting device according to claim 17 **characterised in that** the base portion may be secured to a shoe of a user.

17. A correcting device according to claim 18 **characterised in that** the base portion comprises a pair of base plates disposed on either side of the foot of the user, projection members being pivotally attached thereto and extending around the rear of the wearer's lower leg to act thereon.

18. A correcting device according to claim 19 **characterised in that** the projection members are joined together behind the wearer's lower leg.

19. A correction device according to any of the preceding claims **characterised in that** the first, second and third locations are in alignment along substantially the same axis when the projection member and resilient member are aligned.

## Patentansprüche

1. Korrekturvorrichtung zum Korrigieren der Lage eines Körperteils eines Benutzer mit Bezug auf einen Bereich dieses Benutzers, an dem der genannte Körperteil an einem Gelenk angebracht ist, wobei die genannte Vorrichtung Folgendes umfasst:
einen Basisteil (20, 60) mit Mitteln (22, 62) zum Befestigen der Vorrichtung an dem Körperteil (26) oder dem Benutzerbereich auf einer Seite des Gelenks,
ein Fortsatzelement (24, 24a, 68), das auf dem genannten Basisteil an einer ersten Stelle (25, 70) angelenkt ist und Mittel (21, 56, 74) hat, die auf das Körperteil oder den Benutzerbereich auf der anderen Seite des Gelenks wirken,
und ein federndes Element (34, 34a, 34b) mit einem ersten Ende, das auf dem Basisteil an einer zweiten Stelle (36), die von der ersten Stelle (25, 70) entfernt ist, angebracht ist, und einem zweiten Ende, das an dem Fortsatzelement an einer dritten Stelle (30) angebracht ist, wobei die Vorrichtung dergestalt ist, dass die Drehung des Fortsatzelements von der zweiten Stelle weg die Streckung des federnden Elements vergrößert, wodurch die Größe einer Kraft auf dem genannten Fortsatzelement zum Rückstellen des genannten Fortsatzelements wirkt, während gleichzeitig der zwischen dem federnden Element und dem Fortsatzelement an der dritten Stelle liegende Winkel (θ°) verkleinert wird, sodass eine größere Komponente der Rückstellkraft durch das Fortsatzelement zurück zur Basis übertragen wird und eine kleinere Komponente der genannten Kraft zum Korrigieren der Lage des Körperteils mit Bezug auf den Benutzerbereich wirkt, **dadurch gekennzeichnet, dass** das Fortsatzelement von der zweiten Stelle weg in eine Stellung gedreht werden kann, in der das genannte Fortsatzelement und das federnde Element aufeinander ausgerichtet sind und die auf das genannte Fortsatzelement wirkende Rückstellkraft des federnden Elements völlig von dem genannten Fortsatzelement getragen wird, wodurch die zum Korrigieren der Lage des Körperteils mit Bezug auf den Benutzerbereich wirkende Kraft minimiert wird.

2. Korrekturvorrichtung nach Anspruch 1, **dadurch gekennzeichnet, dass** der Basisteil an dem Benutzerbereich befestigt ist, an dem das Körperteil angebracht ist.

3. Korrekturvorrichtung nach Anspruch 1, **dadurch gekennzeichnet, dass** der Basisteil am Körperteil befestigt ist, und dadurch, dass das Fortsatzelement Mittel hat, die auf den Benutzerbereich wirken, an dem das Körperteil am Gelenk angebracht ist.

4. Korrekturvorrichtung nach Anspruch 1, **dadurch gekennzeichnet, dass** die Schwenkbewegung des Fortsatzelements weg von der zweiten Stelle durch Anschlagmittel zum Verhindern weiterer Drehung des genannten Fortsatzelements über die Stellung hinaus begrenzt ist, in der es axial auf das federnde Element ausgerichtet ist.

5. Korrekturvorrichtung nach Anspruch 1, **dadurch gekennzeichnet, dass** das Fortsatzelement wenigstens zwei aufrechte Abschnitte umfasst, die an einem freien Ende davon verbunden sind.

6. Korrekturvorrichtung nach Anspruch 1, **dadurch gekennzeichnet, dass** das zweite Ende des federnden Elements an der dritten Stelle am oder eng am anderen Ende des Fortsatzelements zu dem befestigt ist, an dem es an der Basis angebracht ist.

7. Korrekturvorrichtung nach Anspruch 1, **dadurch gekennzeichnet, dass** das erste Ende des federnden Elements einstellbar am Basisteil befestigt ist, um eine Einstellung der Spannung des federnden Elements zuzulassen.

8. Korrekturvorrichtung nach einem der vorhergehenden Ansprüche, **dadurch gekennzeichnet, dass** die erste Stelle nahe am Gelenk ist.

9. Korrekturvorrichtung nach einem der vorhergehenden Ansprüche, **dadurch gekennzeichnet, dass** die Vorrichtung eine Mehrzahl von Fortsatzelementen und assoziierten federnden Elementen aufweist und auf einen Benutzer anwendbar ist zur Korrektur der Lage einer Mehrzahl von Fingern mit Bezug auf eine Hand, an der die genannten Finger an Knöcheln angebracht sind.

10. Korrekturvorrichtung nach Anspruch 9, **dadurch gekennzeichnet, dass** das Fortsatzelement mit einer Schlinge versehen ist, in der ein Finger angeordnet werden kann.

11. Korrekturvorrichtung nach einem der Ansprüche 1 oder 3 bis 8, **dadurch gekennzeichnet, dass** das Fortsatzelement mit einer Schlinge versehen ist, in der eine Hand angeordnet werden kann, wobei der genannte Basisteil an einem Unterarm des Trägers befestigt ist, an dem die genannte Hand an einem Handgelenk angebracht ist.

12. Korrekturvorrichtung nach einem der vorhergehenden Ansprüche, **dadurch gekennzeichnet, dass** das federnde Element eine Schraubenfeder ist.

13. Korrekturvorrichtung nach einem der Ansprüche 9 - 10, **dadurch gekennzeichnet, dass** das Fortsatzelement einen Extensionsabschnitt hat, der sich von einem freien Ende davon und weg von dem federnden Element erstreckt.

14. Korrekturvorrichtung nach Anspruch 1, **dadurch gekennzeichnet, dass** der Basisteil mit Bändern am Benutzer befestigt wird, an denen Klettverschlüsse angebracht sind.

15. Korrekturvorrichtung nach Anspruch 1, **dadurch gekennzeichnet, dass** die Vorrichtung eine Korrekturvorrichtung für einen Fuß ist, wobei der genannte Basisteil an dem Fuß, der am Fußgelenk am Bein eines Trägers angebracht ist, befestigt ist und das Fortsatzelement an der Basis angelenkt ist, um auf das genannte Bein zu wirken, um die Lage des genannten Fußes mit Bezug auf das genannte Bein zu korrigieren.

16. Korrekturvorrichtung nach Anspruch 17, **dadurch gekennzeichnet, dass** der Basisteil an einem Schuh eines Benutzers befestigt werden kann.

17. Korrekturvorrichtung nach Anspruch 18, **dadurch gekennzeichnet, dass** der Basisteil ein Paar Basisplatten umfasst, die auf jeder Seite des Fußes des Benutzers angeordnet sind, wobei Fortsatzelemente daran angelenkt sind und sich um die Rückseite des Unterschenkels des Trägers herum erstrecken.

18. Korrekturvorrichtung nach Anspruch 19, **dadurch gekennzeichnet, dass** die Fortsatzelemente hinter dem Unterschenkel des Trägers miteinander verbunden sind.

19. Korrekturvorrichtung nach einem der vorhergehenden Ansprüche, **dadurch gekennzeichnet, dass** die erste, zweite und dritte Stelle entlang weitgehend der gleichen Achse fluchten, wenn das Fortsatzelement und das federnde Element axial aufeinander ausgerichtet sind.

## Revendications

1. Un dispositif correcteur pour corriger l'attitude d'un appendice d'un utilisateur relativement à une partie de cet utilisateur à laquelle ledit appendice est attaché à une articulation
ledit dispositif comprenant
une partie de base (20, 60) ayant des moyens (22, 62) pour fixer le dispositif à l'appendice (26), ou à la partie de l'utilisateur d'un côté de l'articulation,
un élément en saillie (24, 24a, 68) attaché de façon pivotante sur ladite partie de base en un premier emplacement (25, 70) et ayant des moyens (31, 56, 74) qui agissent sur l'appendice ou sur la partie de l'utilisateur de l'autre côté de l'articulation
et un élément résilient (34, 34a, 34b) ayant une première extrémité attachée sur la partie de base en un deuxième emplacement (36) distant du premier emplacement (25, 70) et une deuxième extrémité attachée à l'élément en saillie à un troisième emplacement (30) l'agencement étant tel que la rotation de l'élément en saillie en s'écartant du deuxième emplacement augmente l'extension de l'élément résilient, ce qui accroît la grandeur d'une force exercée sur ledit élément en saillie agissant pour rétablir ledit élément en saillie tout en réduisant simultanément l'angle (θ°) sous-tendu entre l'élément résilient et l'élément en saillie au troisième emplacement de sorte à retransmettre à la base une composante plus grande de la force de rétablissement à travers l'élément en saillie et une composante moins grande de ladite force agit pour corriger l'attitude de l'appendice relativement à la partie de l'utilisateur
**caractérisé en ce que**
il est possible de faire tourner l'élément en saillie en l'écartant du deuxième emplacement jusqu'à une position en laquelle ledit élément en saillie et l'élément résilient sont alignés et la force de rétablissement de l'élément résilient agissant sur ledit élément en saillie est portée en sa totalité par ledit élément en saillie, ce qui minimise la force qui agit pour corriger l'attitude de l'appendice relativement à la partie de l'utilisateur.

2. Un dispositif correcteur selon la revendication 1, **caractérisé en ce que** la partie de base est fixée à la partie de l'utilisateur à laquelle est attaché l'appendice.

3. Un dispositif correcteur selon la revendication 1, **caractérisé en ce que** la partie de base est fixée à l'appendice et **en ce que** l'élément en saillie a des moyens qui agissent sur la partie de l'utilisateur à laquelle l'appendice est attaché au niveau de l'articulation.

4. Un dispositif correcteur selon la revendication 1, **caractérisé en ce que** le mouvement de pivot de l'élément en saillie qui s'écarte du deuxième membre est limité par des moyens d'arrêt pour empêcher toute autre rotation dudit élément en saillie au-delà de la position à laquelle il est aligné avec l'élément résilient.

5. Un dispositif correcteur selon la revendication 1, **caractérisé en ce que** l'élément en saillie comprend au moins deux sections verticales qui sont jointes à une extrémité libre de celles-ci.

6. Un dispositif correcteur selon la revendication 1, **caractérisé en ce que** la deuxième extrémité de l'élément résilient est fixée au troisième emplacement au niveau ou à proximité de l'autre extrémité de l'élément en saillie et non à celle à laquelle est attaché la base.

7. Un dispositif correcteur selon la revendication 1, **caractérisé en ce que** la première extrémité de l'élément résilient est fixée de façon ajustable à la partie de base pour rendre possible un ajustement de la tension de l'élément résilient.

8. Un dispositif correcteur selon l'une quelconque des revendications précédentes, **caractérisé en ce que** le premier emplacement est le plus proche de l'articulation.

9. Un dispositif correcteur selon l'une quelconque des revendications précédentes, **caractérisé en ce que** le dispositif comprend une pluralité d'éléments en saillie et d'éléments résilients associés, et est applicable à un utilisateur pour corriger les attitudes d'une pluralité de doigts relativement à une main à laquelle lesdits doigts sont attachés au niveau des jointures des doigts.

10. Un dispositif correcteur selon la revendication 9, **caractérisé en ce que** l'élément en saillie est pourvu d'une écharpe dans laquelle un doigt peut être disposé.

11. Un dispositif correcteur selon l'une quelconque des revendications 1, ou 3 à 8, **caractérisé en ce que** l'élément en saillie est pourvu d'une écharpe dans laquelle une main peut être disposée, ladite partie de base étant fixée à un avant-bras du porteur auquel ladite main est attachée au niveau d'un poignet.

12. Un dispositif correcteur selon l'une quelconque des revendications précédentes, **caractérisé en ce que** l'élément résilient est un ressort à boudin.

13. Un dispositif correcteur selon l'une ou l'autre des revendications 9 ou 10, **caractérisé en ce que** l'élément en saillie comprend une section d'extension qui se prolonge depuis une extrémité libre de celle-ci en s'écartant de l'élément résilient.

14. Un dispositif correcteur selon la revendication 1, **caractérisé en ce que** la partie de base est fixée à l'utilisateur avec des sangles, qui portent des fixations de type Velcro.

15. Un dispositif correcteur selon la revendication 1, **caractérisé en ce que** le dispositif est un dispositif de correction pour un pied, ladite partie de base étant fixée au pied attaché à la jambe d'un porteur au niveau de la cheville et l'élément en saillie étant attaché de façon pivotante à la base pour agir sur ladite jambe pour corriger l'attitude dudit pied relativement à ladite jambe.

16. Un dispositif correcteur selon la revendication 17, **caractérisé en ce que** la partie de base peut être fixée à une chaussure d'un utilisateur.

17. Un dispositif correcteur selon la revendication 18, **caractérisé en ce que** la partie de base comprend une paire de plaques de base disposées de chaque côté du pied de l'utilisateur, les éléments en saillie étant attachés de façon pivotante à celles-ci et s'étendant autour de l'arrière de la partie inférieure de la jambe du porteur de sorte à agir sur celle-ci.

18. Un dispositif correcteur selon la revendication 19, **caractérisé en ce que** les éléments en saillie sont joints ensemble derrière la partie inférieure de la jambe du porteur.

19. Un dispositif correcteur selon l'une quelconque des revendications précédentes, **caractérisé en ce que** les premier, deuxième et troisième emplacements sont alignés sensiblement le long d'un même axe lorsque l'élément en saillie et l'élément résilient sont alignés.
